# FASCICULE DE BREVET EUROPEEN

(11) **EP 2 736 541 B1**
(45) Date de publication et mention de la délivrance du brevet: **30.12.2020**
(21) Numéro de dépôt: 12744098.0
(22) Date de dépôt: 24.07.2012
(51) Int. Cl.: A61L 11/00, B09B 3/00

(54) **PROCEDE ET DISPOSITIF DE TRAITEMENT THERMIQUE DE DECHETS**
VERFAHREN UND VORRICHTUNG ZUR WÄRMEBEHANDLUNG VON ABFALL
METHOD AND APPARATUS FOR HEAT TREATMENT OF WASTE

(30) Priorité: 26.07.2011 FR 1156794
(43) Date de publication de la demande: 04.06.2014
(73) Titulaire: La Société STERIGERMS FRANCE, 26600 La Roche de Glun (FR)
(72) Inventeur: BERAUD Christophe, 07630 Le Béage (FR)
(74) Mandataire: Chevalier, Renaud Philippe
(86) Numéro de dépôt international: PCT/FR2012/051753
(87) Numéro de publication internationale: WO 2013/014387

(56) Documents cités:
- WO-A1-99/11299
- GB-A- 2 465 144
- US-B1- 7 204 956

## Description

La présente invention a trait à un procédé de traitement thermique de déchets ainsi qu'à un dispositif pour mettra en œuvre ce procédé.

Cette invention concerne le domaine du traitement des déchets, plus particulièrement celui du traitement de ces déchets par la chaleur et à l'intérieur d'une enceinte chauffée. Cette invention trouvera une application toute particulière mais aucunement limitative lorsqu'il s'agit de traiter des déchets ménagers, hospitaliers ou à risques biologiques.

L'on connaît, d'ores et déjà, des procédés de traitement de déchets de ce type et consistant à introduire des déchets dans un sac avant de positionner, à l'intérieur d'un dispositif conçu pour traiter ces déchets, une charge constituée par ce sac et par les déchets contenus dans ce sac.

Le procédé consiste, alors et après avoir introduit la charge à l'intérieur du dispositif, à assurer une montée en température du fond et du couvercle et, ensuite, à faire observer un palier de température à ce fond et à ce couvercle, donc à la charge. De plus, avant d'atteindre le palier de température, il est procédé à au moins un rapprochement entre le fond et le couvercle, voire à un compactage de la charge entre ce fond et ce couvercle.

Pour la mise en œuvre de ce procédé, il est fait appel à un dispositif, notamment tel décrit dans le document US-7.204.956, comportant un fond et un couvercle incorporant des moyens de chauffage qui sont à l'origine d'une inhomogénéité de température à l'intérieur de la charge. Cette inhomogénéité conduit à un traitement inhomogène et/ou incomplet des déchets (ce qui n'est pas admissible pour des déchets hospitaliers ou à risques biologiques) ou nécessite, pour un traitement approprié de ces déchets, un allongement substantiel de la durée de mise en œuvre du procédé ce qui se traduit par une consommation d'énergie considérable.

De plus, ce procédé consiste à assurer un rapprochement du fond et du couvercle, voire une compression de la charge. Or, lorsque la charge contient un objet non compactable, le rapprochement du fond et du couvercle est interrompu et la charge est directement soumise au palier de température. L'interruption de ce rapprochement engendre une insuffisance de compactage de la charge ce qui conduit, là encore, à une inhomogénéité de la température à l'intérieur de cette charge avec les mêmes conséquences que mentionné ci-dessus.

En outre et malgré un rapprochement du fond et du couvercle, la charge contient, habituellement, une certaine quantité d'air résiduel qui est à l'origine d'une mauvaise conduction de la chaleur et, donc, d'une inhomogénéité de la température à l'intérieur de la charge, là encore avec les mêmes conséquences que mentionné ci-dessus.

Finalement, le procédé consiste à permettre une évacuation de l'air hors du dispositif qui comporte, alors, un filtre conçu pour assurer la filtration de cet air évacué. Or, un tel filtre est particulièrement sensible à la présence, dans l'air évacué, de liquide, notamment d'eau, qui rend ce filtre totalement inactif et nécessite son nettoyage, voire son démontage. Afin de remédier à cet inconvénient, le dispositif est équipé de moyens conçus pour détecter la présence d'un tel liquide et pour interrompre le procédé de traitement des déchets en cas de détection d'un tel liquide. Etant donné que la majorité des déchets contiennent un tel liquide, soit il est procédé rapidement à une interruption du traitement de ces déchets, soit il est procédé à un allongement conséquent de la durée de chauffage de la charge ce qui, là encore, se traduit par une consommation d'énergie considérable.

La présente invention se veut de remédier aux inconvénients des procédés de l'état de la technique consistant à assurer le traitement des déchets ainsi que des dispositifs pour mettre en œuvre ces procédés.

A cet effet, l'invention concerne un procédé de traitement thermique de déchets, ce procédé consistant en ce que :
- on introduit les déchets à traiter dans un sac ;
- on positionne le sac contenant les déchets à l'intérieur d'un dispositif, conçu pour traiter ces déchets, et comportant un fût cylindrique, un couvercle amovible de fermeture de ce fût cylindrique, et un fond mobile à l'intérieur de ce fût cylindrique, ceci au moins en direction du couvercle ;
- après positionnement du sac contenant les déchets à l'intérieur du dispositif :
   - on procède à un traitement thermique de ces déchets, tout d'abord en assurant une montée en température du couvercle et du fond du dispositif et, ensuite, en faisant observer à ce couvercle et à ce fond un palier de température.

Ce procédé est caractérisé par le fait que :
- au moins pendant la montée en température du couvercle et du fond et avant d'atteindre le palier de température, on procède à au moins un rapprochement entre ce couvercle et ce fond et par le fait qu'on confère au couvercle, respectivement au fond, une température qui diffère d'au plus de 2°C de la température du fond, respectivement du couvercle, ceci au moins durant le palier de température.

Un procédé consiste, encore, en ce que, avant d'atteindre le palier de température et après au moins un rapprochement entre le fond et le couvercle, on procède à un éloignement entre ce fond et ce couvercle suivi d'un nouveau rapprochement entre ce fond et ce couvercle.

Une autre caractéristique de ce procédé consiste en ce que, avant de faire observer un palier de température au fond et au couvercle, on mesure la distance entre le fond et le couvercle pour, soit faire observer ledit palier de température lorsque cette distance est inférieure à une valeur déterminée, soit interrompre le procédé lorsque cette distance est supérieure à cette valeur déterminée.

Une caractéristique additionnelle consiste en ce qu'on assure une filtration, par un filtre que comporte le dispositif, de l'air évacué hors du dispositif ainsi qu'un chauffage de ce filtre à une température permettant de vaporiser le liquide contenu dans l'air évacué.

L'invention concerne, également, un dispositif de traitement de déchets comportant une enceinte, destinée à recevoir les déchets à traiter, et comportant un fût cylindrique, un couvercle amovible de fermeture de ce fût cylindrique, et un fond mobile à l'intérieur de ce fût cylindrique, ceci au moins en direction du couvercle.

Ce dispositif de traitement est caractérisé par le fait qu'il comporte des moyens pour conférer au couvercle, respectivement au fond, une température déterminée qui diffère d'au plus de 2°C de la température du fond, respectivement du couvercle, les moyens pour conférer une température déterminée au couvercle, respectivement au fond, comportent :
- au moins un moyen pour relever au moins la température de ce couvercle, respectivement de ce fond;
- au moins un moyen pour chauffer ce couvercle, respectivement ce fond;
- au moins un moyen pour refroidir ce couvercle, respectivement de ce fond;
- des moyens pour piloter le moyen pour chauffer ce couvercle, respectivement ce fond, ainsi que le moyen pour refroidir ce couvercle, respectivement ce fond, ceci au moins en fonction de la température déterminée et de la température relevée de ce couvercle, respectivement de ce fond.

Ainsi, le procédé conforme à l'invention consiste à conférer au fond, respectivement au couvercle, une température qui diffère d'au plus de 2°C de la température du couvercle, respectivement du fond, ceci au moins durant le palier de température. Une telle caractéristique permet, avantageusement, d'établir une température homogène au sein de la charge ce qui conduit à un traitement homogène et complet des déchets.

De plus et de manière particulièrement surprenante, le fait de conférer au fond, respectivement au couvercle, une température qui diffère d'au plus de 2°C de la température du couvercle, respectivement du fond, ceci au moins durant le palier de température, permet de diminuer de manière considérable la durée de ce palier de température et, par conséquent, la durée du cycle de traitement des déchets qui est au moins divisé par quatre.

De plus, avant d'atteindre le palier de température et après au moins un rapprochement entre le fond et le couvercle, le procédé consiste à assurer un éloignement entre ce fond et ce couvercle suivi d'un nouveau rapprochement entre ce fond et ce couvercle. Cet éloignement/rapprochement permet, avantageusement, de s'assurer qu'il n'y plus d'air dans la charge ce qui contribue à établir une température homogène au sein de la charge.

Encore une autre caractéristique consiste en ce que, avant de faire observer un palier de température au fond et au couvercle, on mesure la distance entre le fond et le couvercle pour, soit faire observer ledit palier de température lorsque cette distance est inférieure à une valeur déterminée, soit interrompre le procédé lorsque cette distance est supérieure à cette valeur déterminée. Cette caractéristique permet, avantageusement, d'assurer le traitement des déchets uniquement lorsque le fond et le couvercle sont suffisamment rapprochés pour garantir une température homogène au sein de la charge.

En outre, on assure une filtration de l'air sortant du dispositif ainsi qu'un chauffage de ce filtre à une température permettant de vaporiser le liquide (plus particulièrement l'eau) contenu dans l'air ce qui permet, avantageusement, d'une part, d'éviter la présence de liquide dans le filtre et, par conséquent, de conserver les propriétés de filtration et l'efficacité du filtre et, d'autre part, d'évacuer un maximum de liquide contenu dans la charge. En évacuant ce liquide, on minimise la quantité de liquide au sein de la charge ce qui permet, avantageusement et en raison de la faible compressibilité d'un tel liquide, d'améliorer le taux de compression de la charge et, par conséquent, de minimiser le volume de la charge. Ceci permet de maximiser le rapprochement du fond et du couvercle et, par conséquent, d'améliorer l'homogénéité de la température au sein de la charge et, ainsi, le traitement des déchets.

Finalement, chacune de ces caractéristiques permet, avantageusement, d'optimiser le traitement des déchets, de raccourcir la durée du traitement des déchets et, ainsi, de réaliser une économie d'énergie substantielle.

D'autres buts et avantages de la présente invention apparaîtront au cours de la description qui va suivre se rapportant à des modes de réalisation qui ne sont donnés qu'à titre d'exemples indicatifs et non limitatifs.

La compréhension de cette description sera facilitée en se référant aux dessins joints en annexe et dans lesquels :

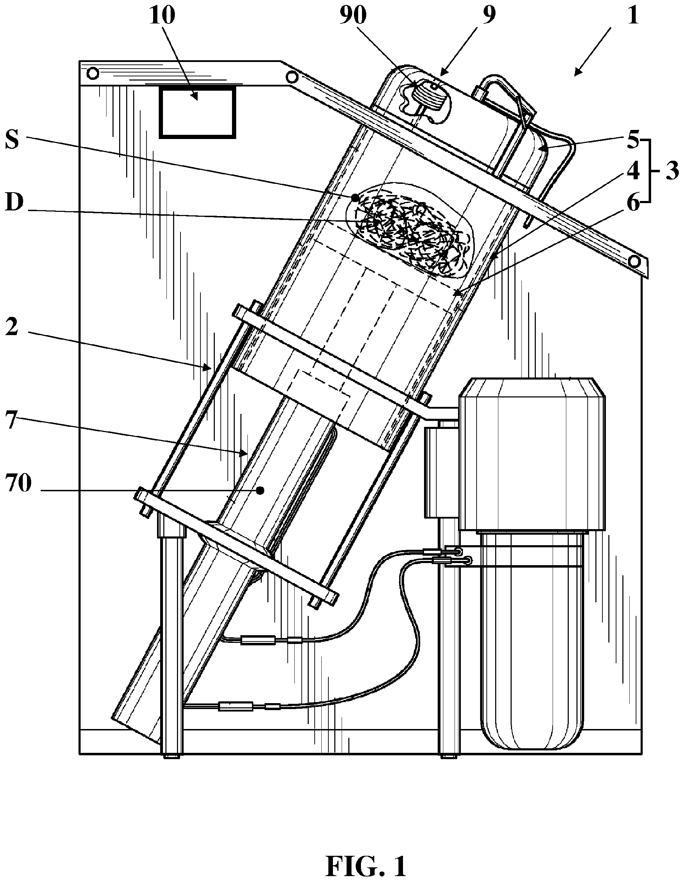

- la figure 1 est une vue schématisée, de côté et en coupe partielle, du dispositif de traitement de déchets conforme à l'invention ;

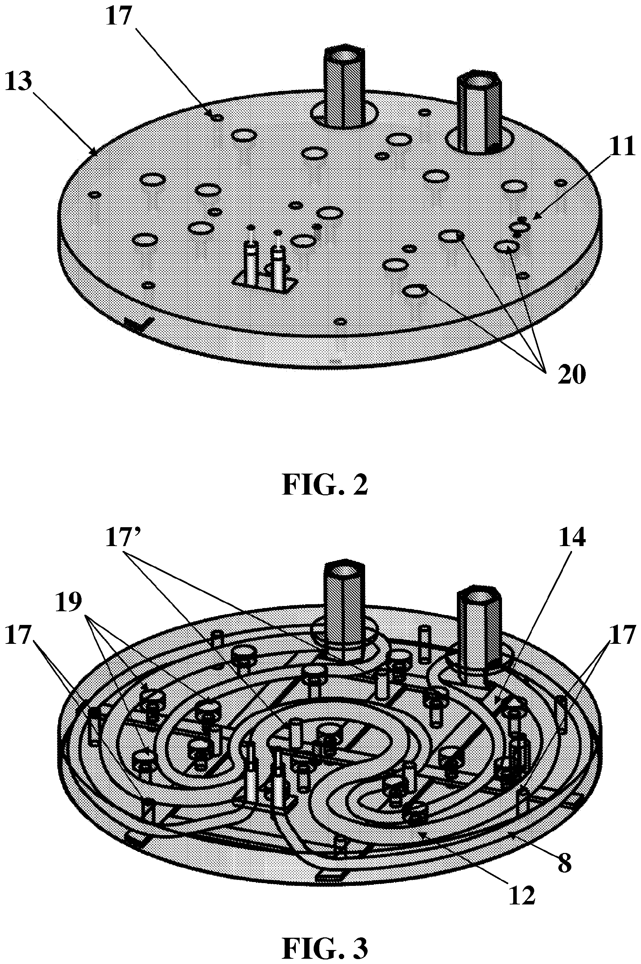

- la figure 2 est une vue schématisée et en perspective d'un bloc thermique que comporte le fond et le couvercle du dispositif illustré figure 1 ;
- la figure 3 est une vue similaire à la figure 2 et correspond à l'agencement des différents éléments que comporte intérieurement le bloc thermique ;

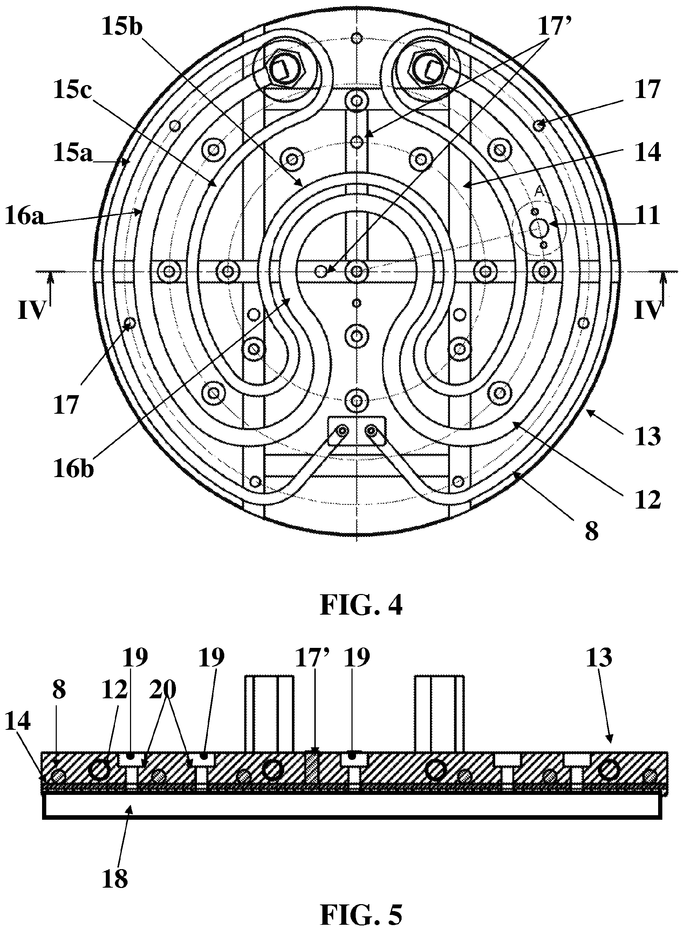

- la figure 4 est une vue schématisée et de dessus de l'agencement des différents éléments que comporte le bloc thermique ;
- la figure 5 est une vue schématisée et en coupe selon IV-IV du bloc thermique illustré figure 4 ;

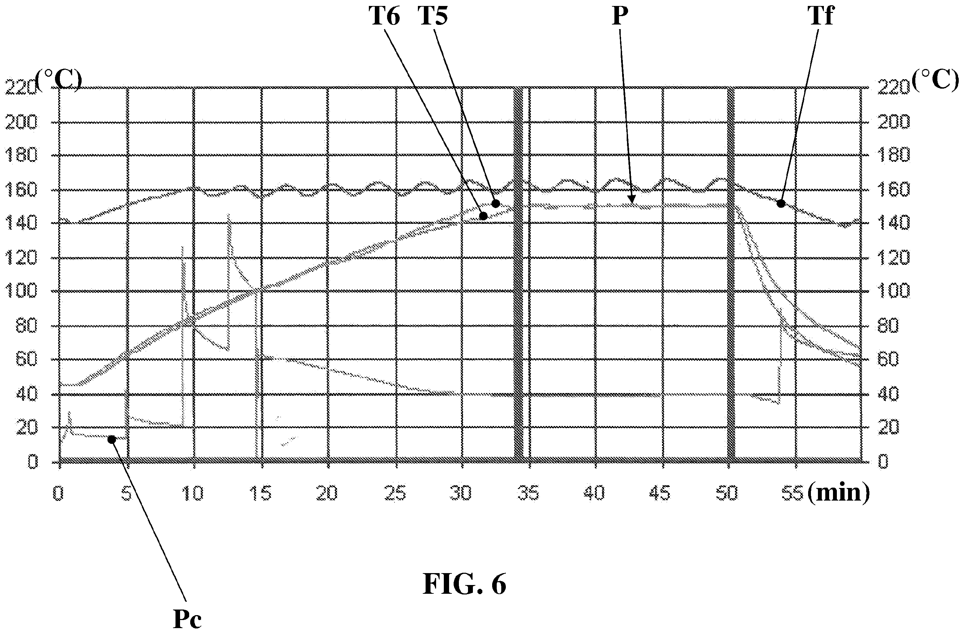

- la figure 6 est un graphique représentant, en fonction du temps, l'évolution de différents paramètres (température du fond, température du couvercle, température du filtre, pression à l'intérieur de la charge) au cours de la mise en œuvre du procédé conforme à l'invention.

La présente invention concerne le domaine du traitement des déchets, plus particulièrement celui du traitement de ces déchets par la chaleur et à l'intérieur d'une enceinte chauffée.

L'invention concerne, alors, un procédé de traitement thermique (plus particulièrement de désinfection) de déchets, notamment de déchets à risques biologiques, hospitaliers ou ménagers.

L'invention concerne, également, un dispositif 1 de traitement de déchets D, plus particulièrement conçu pour mettre en œuvre le procédé de traitement mentionné ci-dessus.

De manière connue, un dispositif 1 de traitement des déchets D comporte un châssis 2 ainsi qu'une enceinte 3, montée sur ce châssis 2, et à l'intérieur de laquelle est réalisé le traitement des déchets D.

Cette enceinte 3 comporte un fût cylindrique 4, délimité par une paroi interne, et s'étendant selon un axe formant un angle de préférence compris entre 45 et 90° par rapport à l'horizontale.

Cette enceinte 3 comporte, également, un couvercle 5 pour la fermeture du fût cylindrique 4 et, par conséquent, de cette enceinte 3. Ce couvercle 5 est de type amovible pour permettre l'accès à l'intérieur du fût cylindrique 4 et est monté de manière articulée par rapport au châssis 2, ceci entre une position d'ouverture et une position de fermeture de ce fût 4.

Cette enceinte 3 comporte, également, un fond 6, positionné à l'intérieur du fût cylindrique 4, et mobile en déplacement à l'intérieur de ce fût cylindrique 4, ceci au moins en direction du couvercle 5.

Le dispositif 1 de traitement des déchets D comporte, encore, des moyens 7 pour entraîner en déplacement le fond 6 de l'enceinte 3.

De tels moyens d'entraînement 7 comportent un vérin 70 interposé entre ce fond 6 et le châssis 2. Ce vérin 70 peut être de type électrique, pneumatique ou, et de préférence, hydraulique. Dans le cas d'un vérin 70 de type pneumatique ou hydraulique, le dispositif 1 comporte, encore (plus particulièrement implantés sur le châssis 2), des moyens pour l'alimentation en fluide (pneumatique ou hydraulique) de ce vérin 70.

Ce dispositif de traitement 1 comporte, également, des moyens 8 pour chauffer le couvercle 5 ainsi que des moyens 8 pour chauffer le fond 6.

De plus, ce dispositif de traitement 1 comporte des moyens 9 pour évacuer l'air en dehors de l'enceinte 3.

Ces moyens 9 pour évacuer l'air comportent une vanne, plus particulièrement une électrovanne, conçue pour fermer ces moyens 9 pour évacuer l'air et, donc, pour empêcher l'évacuation de l'air, ceci plus particulièrement en cas de détection de liquide (notamment de l'eau) au cours du déroulement d'un procédé de traitement des déchets D.

De plus, ces moyens 9 pour évacuer l'air comportent un filtre 90 conçu pour filtrer l'air sortant de l'enceinte 3. Un tel filtre 90 est, de préférence, de type hydrophobe.

Finalement, le dispositif 1 comporte des moyens 10 pour piloter le fonctionnement de ce dispositif 1, plus particulièrement au moins les moyens 8 pour chauffer le couvercle 5 et le fond 6 ainsi que les moyens 7 pour entraîner en déplacement ce fond 6.

Ces moyens 10 pour piloter le dispositif 1 comportent un moyen de traitement (plus particulièrement sous forme d'un logiciel) conçu pour faire exécuter à ce dispositif 1 une succession d'étapes d'un procédé de traitement des déchets D.

Ces moyens 10 pour piloter le dispositif 1 comportent, encore, une mémoire incorporant au moins une succession d'étapes correspondant à au moins un procédé de traitement de déchets D.

Finalement, ces moyens 10 pour piloter le dispositif 1 comportent une interface au niveau de laquelle un utilisateur du dispositif 1 commande l'exécution des différentes étapes d'un tel procédé de traitement de déchets D et/ou sélectionne les paramètres de ces différentes étapes.

Selon l'invention, ce dispositif comporte des moyens pour conférer au couvercle 5, respectivement au fond 6, une température déterminée qui diffère d'au plus de 2°C de la température du fond 6, respectivement du couvercle 5.

En fait, ces moyens sont, de préférence, conçus pour conférer à ce couvercle 5, respectivement à ce fond 6, une température déterminée qui diffère d'au plus de 1°C de la température du fond 6, respectivement du couvercle 5.

A ce propos, il convient d'observer que les moyens pour conférer une température déterminée au couvercle 5, respectivement au fond 6, comportent au moins un moyen 11 pour relever au moins la température de ce couvercle 5, respectivement de ce fond 6.

Selon un mode particulier de réalisation, ces moyens pour conférer une température déterminée au couvercle 5, respectivement au fond 6, comportent, encore, au moins un moyen 11 pour relever au moins la température du fond 6, respectivement la température du couvercle 5.

Un tel moyen 11 pour relever la température est, de préférence, constitué par une sonde ou analogue.

Les moyens pour conférer une température déterminée au couvercle 5, respectivement au fond 6, comportent, encore, au moins un moyen 8 pour chauffer ce couvercle 5, respectivement ce fond 6.

Un tel moyen 8 pour chauffer est, plus particulièrement, constitué par au moins une résistance, notamment de type électrique.

Les moyens pour conférer une température déterminée au couvercle 5, respectivement au fond 6, comportent, également, au moins un moyen 12 pour refroidir ce couvercle 5, respectivement ce fond 6.

En fait, un tel moyen 12 pour refroidir est, plus particulièrement, constitué par une conduite à l'intérieur de laquelle circule un fluide caloriporteur.

Finalement, les moyens pour conférer une température déterminée au couvercle 5, respectivement au fond 6, comportent des moyens 10 pour piloter le moyen 8 pour chauffer ce couvercle 5, respectivement ce fond 6, ainsi que le moyen 12 pour refroidir ce couvercle 5, respectivement ce fond 6, ceci au moins en fonction de la température déterminée et de la température relevée de ce couvercle 5, respectivement de ce fond 6.

Selon un mode particulier de réalisation, ces moyens 10 pour piloter le moyen S pour chauffer ce couvercle 5, respectivement ce fond 6, ainsi que le moyen 12 pour refroidir ce couvercle 5, respectivement ce fond 6, peuvent, de manière alternative ou complémentaire, être conçus pour piloter ces moyens (8 ; 12) en fonction de la température déterminée et de la température relevée du fond 6, respectivement du couvercle 5.

Pour ce faire, les moyens pour conférer une température déterminée au couvercle 5, respectivement au fond 6, comportent au moins un moyen pour relever la température du fond 6, respectivement du couvercle 5.

Dans un pareil cas, la température déterminée peut être constituée par la température relevée du fond 6, respectivement du couvercle 5.

Tel que mentionné ci-dessus, les moyens pour conférer une température déterminée au couvercle 5, respectivement au fond 6, comportent, d'une part, au moins un moyen 8 pour chauffer ce couvercle 5, respectivement ce fond 6, et, d'autre part, au moins un moyen 12 pour refroidir ce couvercle 5, respectivement ce fond 6.

La présence d'un tel moyen pour chauffer 8 et d'un tel moyen 12 pour refroidir au niveau du couvercle 5, respectivement du fond 6, permet, avantageusement, de créer un effet de synergie entre ces moyens (8 ; 12) conduisant à une régulation particulièrement précise de la température, à une précision accrue de la température (du couvercle 5, respectivement du fond 6) ainsi qu'à une exactitude de la température (du couvercle 5, respectivement de ce fond 6), ceci par rapport à la température déterminée.

Selon une autre caractéristique de l'invention, le couvercle 5, respectivement le fond 6, comporte un bloc thermique 13 incorporant au moins un moyen 8 pour chauffer ce couvercle 5, respectivement ce fond 6, ainsi qu'au moins un moyen 12 pour refroidir ce couvercle 5, respectivement de ce fond 6.

En fait, ce bloc thermique 13 est, de préférence, réalisé par coulage d'un matériau.

Dans un pareil cas, au moins un moyen 8 pour chauffer et au moins un moyen 12 pour refroidir sont au moins en partie noyés dans le matériau de ce bloc thermique 13 qui présente une température de fusion inférieure à celle d'un tel moyen 8 pour chauffer et à celle d'un tel moyen 12 pour refroidir.

A ce propos, il convient d'observer que, selon un mode particulier de réalisation de l'invention, le matériau du bloc thermique 13 est constitué par de l'aluminium, le matériau d'un moyen 8 pour chauffer (plus particulièrement une résistance) est constitué par du cuivre tandis que le matériau d'un moyen 12 pour refroidir (plus particulièrement une conduite de fluide caloriporteur) est constitué par un alliage chrome molybdène.

Une autre caractéristique consiste en ce que le bloc thermique 13 comporte une armature 14, noyée dans le matériau du bloc thermique 13, et sur laquelle 14 reposent et/ou de laquelle 14 sont rendus solidaires, au moins un moyen 8 pour chauffer et/ou au moins un moyen 12 pour refroidir.

La présence d'une telle armature 14 et son association avec un tel moyen 8 pour chauffer et/ou avec un tel moyen 12 pour refroidir permet, avantageusement, d'assurer un positionnement approprié de ces moyens (8 ; 12) au sein du bloc thermique 13, ceci lors de la fabrication de celui-ci 13, plus particulièrement au moment du coulage du matériau de ce bloc thermique 13.

Encore une autre caractéristique consiste en ce que le moyen 8 pour chauffer, respectivement le moyen 12 pour refroidir, comporte au moins une portion interposée entre deux portions du moyen 12 pour refroidir, respectivement du moyen 8 pour chauffer.

Un tel mode de réalisation permet, avantageusement, de réguler de manière optimale la température à proximité d'une telle portion de moyen 8 pour chauffer, respectivement de moyen 12 pour refroidir.

Selon un mode préféré de réalisation, le moyen 8 pour chauffer et le moyen 12 pour refroidir s'étendent sensiblement dans un plan et sont conformés en sorte de définir au moins une boucle (15 ; 16). Dans un pareil cas, au moins une partie d'une boucle 15 du moyen 8 pour chauffer, respectivement au moins une partie d'une boucle 16 du moyen 12 pour refroidir, est inscrite à l'intérieur d'au moins une partie d'une boucle (16 ; 15) du moyen 12 pour refroidir, respectivement du moyen 8 pour chauffer.

Plus particulièrement, le moyen 8 pour chauffer et le moyen 12 pour refroidir s'étendent sensiblement dans un plan et sont conformés en sorte de définir au moins une boucle externe (15a ; 16a) ainsi qu'au moins une boucle interne (15b ; 16b) raccordée à la boucle externe (15a ; 16a). Dans un pareil cas, une boucle 15 du moyen 8 pour chauffer, respectivement une boucle 16 du moyen 12 pour refroidir, est interposée entre une boucle externe (16a ; 15a) et une boucle interne (16b ; 15b) du moyen 12 pour refroidir, respectivement du moyen 8 pour chauffer.

Tel que visible sur les figures en annexe, le moyen 8 pour chauffer s'étend sensiblement dans un plan et est conformé en sorte de définir une boucle externe 15a, une boucle interne 15b, et une boucle intermédiaire 15c raccordée à la boucle externe 15a et à la boucle interne 15b,

Quant au moyen 12 pour refroidir, celui-ci comporte, d'une part, une boucle externe 16a interposée entre les boucles externe 15a et intermédiaire 15c du moyen 8 pour chauffer et, d'autre part, une boucle interne 16b inscrite à l'intérieur de la boucle interne 15b de ce moyen 8 pour chauffer.

Une caractéristique additionnelle consiste en ce que le bloc thermique 13 comporte des moyens 17 pour positionner au moins un moyen 8 pour chauffer, respectivement au moins un moyen 12 pour refroidir, ceci par rapport à l'armature 14 et/ou par rapport à au moins un moyen 12 pour refroidir, respectivement par rapport à au moins un moyen 8 pour chauffer.

Ce bloc thermique 13 peut, également, comporter des moyens 17' pour positionner ladite armature 14, ceci à l'intérieur d'un moule dans lequel est coulé le matériau destiné à constituer ledit bloc thermique 13.

En fait, ces moyens (17 ; 17') de positionnement sont, plus particulièrement, conçus pour assurer le positionnement d'une armature 14, d'un moyen 8 pour chauffer et/ou d'un moyen 12 pour refroidir, ceci à l'intérieur d'un moule dans lequel est coulé le matériau destiné à constituer ledit bloc thermique 13 et dans la position définitive qu'ils adoptent au sein de ce bloc thermique 13.

Tel que visible sur les figures en annexe, ces moyens (17 ; 17') pour positionner adoptent la forme d'un plot ou analogue qui peut être rendu solidaire de l'armature 14 et s'étendre à partir de cette armature 14, notamment de manière perpendiculaire.

Selon une autre caractéristique de l'invention, le couvercle 5, respectivement le fond 6, comporte une plaque 18 en matériau inoxydable, positionnée à l'intérieur du fut cylindrique 4, comportant une face interne orientée en direction du fond 6, respectivement du couvercle 5, ainsi qu'une face externe, orientée, d'une part, dans une direction opposée à celle du fond 6, respectivement du couvercle 5, et, d'autre part, en direction du bloc thermique 13 que comporte ce couvercle 5, respectivement ce fond 6, et qui est rendu solidaire de cette plaque 18 en matériau inoxydable.

Selon une caractéristique additionnelle, la plaque 18 inoxydable que comporte le fond 6 présente des dimensions (plus particulièrement un diamètre) qui, par rapport aux dimensions internes du fût cylindrique 4, sont définies en sorte d'autoriser un déplacement sans frottement de ce fond 6 à l'intérieur de ce fût cylindrique 4.

Une autre caractéristique consiste en ce que le couvercle 5, respectivement le fond 6, comporte des moyens 19 pour la fixation de la plaque 18 en matériau inoxydable sur le bloc thermique 13.

En fait, de tels moyens de fixation 19 sont constitués par des vis, traversant une ouverture 20 que comporte le bloc thermique 13, et coopérant avec un taraudage que comporte la plaque 18 en matériau inoxydable.

Une caractéristique additionnelle consiste en ce que le couvercle 5, respectivement le fond 6, comporte des moyens pour régler la position de cette plaque 18 en matériau inoxydable par rapport au bloc thermique 13.

La présence de ces moyens de régalage permet, avantageusement, d'optimiser la planéité de la plaque 18 en matériau inoxydable.

De manière additionnelle, le couvercle 5, respectivement le fond 6, comporte des moyens pour établir un pont thermique entre la plaque 18 en matériau inoxydable et le bloc thermique 13.

A ce propos, on observera que les moyens de réglage et/ou les moyens pour établir un pont thermique peuvent, au moins en partie, être constitués par les moyens 19 pour la fixation de la plaque 18 en matériau inoxydable sur le bloc thermique 13.

Selon une autre caractéristique, le dispositif 1 de traitement comporte, encore, des moyens pour guider le fond 6 en translation par rapport au fût cylindrique 4.

La présence de ces moyens de guidage permet, avantageusement, d'assurer un déplacement de ce fond 6 à l'intérieur de ce fût 4 uniquement par translation et, ainsi, d'empêcher la rotation du fond 6 par rapport au fût 4 à l'origine de blocage de ce fond 6.

En fait, selon un mode préféré de réalisation, ces moyens de guidage comportent, d'une part, une rainure fixe associée au fût cylindrique 4 et, d'autre part, ergot mobile, associé au fond 6, et coopérant avec ladite rainure fixe.

Finalement, le dispositif 1 de traitement comporte des moyens pour déterminer la distance entre le fond 6 et le couvercle 5.

Ces moyens pour déterminer une telle distance sont, en fait, constitués par des moyens pour mesurer la distance entre ce fond 6 et une référence fixe, que comporte le dispositif 1, et qui est située à une distance fixe du couvercle 5.

En fait et selon un mode de réalisation préféré de l'invention, ces moyens de mesure comportent, d'une part, un fil rendu solidaire du fond 6, d'autre part, un moyen pour enrouler ce fil (plus particulièrement sous forme d'une bobine) et, d'autre part encore, un moyen pour mesurer la longueur du fil enroulé/déroulé (plus particulièrement sous forme d'un moyen pour compter le nombre de tour de bobine).

Tel que mentionné ci-dessus, la présente invention concerne, également, un procédé pour le traitement thermique de déchets D, notamment ménagers, hospitaliers ou à risques biologiques.

Ce procédé est, plus particulièrement (mais pas exclusivement), mis en œuvre par le dispositif de traitement 1 décrit ci-dessus lequel est, plus particulièrement (mais pas exclusivement), conçu pour mettre en œuvre ce procédé.

En fait, ce procédé de traitement thermique de déchets D consiste en ce que :
- on introduit les déchets D à traiter dans un sac S ;
- on positionne le sac S contenant les déchets D à l'intérieur d'un dispositif 1, conçu pour traiter ces déchets D, et comportant un fût cylindrique 4, un couvercle 5 amovible de fermeture de ce fût cylindrique 4, et un fond mobile 6 à l'intérieur de ce fût cylindrique 4, ceci au moins en direction du couvercle 5.

En fait, ce procédé consiste, plus particulièrement, à positionner le sac S contenant les déchets D à l'intérieur d'une enceinte 3, que comporte ce dispositif 1, et qui comporte un tel fût cylindrique 4, un tel couvercle 4 et un tel fond mobile 6.

Ce procédé consiste, également, en ce que, après positionnement du sac S les déchets D à l'intérieur du dispositif 1 :
- on procède à un traitement thermique de ces déchets D, tout d'abord en assurant une montée en température du couvercle 5 et du fond 6 du dispositif 1 et, ensuite, en faisant observer à ce couvercle 5 et à ce fond 6 un palier P de température ;
- au moins pendant (voire encore avant) la montée en température du couvercle 5 et du fond 6 et avant d'atteindre le palier P de température, on procède à au moins un rapprochement entre ce couvercle 5 et ce fond 6.

Selon l'invention, ce procédé consiste en ce qu'on confère au couvercle 5, respectivement au fond 6, une température (T5, respectivement T6) qui diffère d'au plus de 2°C de la température (T6, respectivement T5) du fond 6, respectivement du couvercle 5, ceci au moins durant le palier de température.

En fait, ce procédé consiste, de préférence, en ce qu'on confère à ce couvercle 5, respectivement à ce fond 6, une température (T5, respectivement T6) qui diffère d'au plus de 1°C de la température (T6, respectivement T5) du fond 6, respectivement du couvercle 5, ceci au moins durant le palier de température.

De préférence, ce procédé consiste en ce qu'on confère au couvercle 5, respectivement au fond 6, une température qui diffère d'au plus de 2°C (de préférence d'au plus de 1°C) de la température du fond 6, respectivement du couvercle 5, ceci durant le palier P de température ainsi que pendant la montée en température du couvercle 5 et du fond 6.

Selon une autre caractéristique de ce procédé, au moins pendant (voire encore avant) la montée en température du couvercle 5 et du fond 6 et avant d'atteindre le palier P de température, on procède à une pluralité de rapprochements successifs entre le fond 6 et le couvercle 5, ceci sans procéder à un éloignement entre le fond 6 et le couvercle 5 entre deux rapprochements successifs.

En fait, on procède à au moins un rapprochement entre le fond 6 et le couvercle 5, ceci avec compression du sac S et des déchets D. Un tel mode de réalisation permet de réduire le volume du sac S et des déchets D ainsi que d'évacuer l'air y contenu.

Selon une autre caractéristique, préalablement à un rapprochement avec compression, on procède à au moins un rapprochement entre le fond 6 et le couvercle 5, ceci sans compression du sac S et des déchets D. Un tel mode de réalisation permet d'évacuer l'air à l'extérieur de l'enceinte 3.

Une caractéristique additionnelle de ce procédé consiste en ce que, avant d'atteindre le palier P de température et après au moins un rapprochement entre le fond 6 et le couvercle 5, on procède à un éloignement entre ce fond 6 et ce couvercle 5 suivi d'un nouveau rapprochement entre ce fond 6 et ce couvercle 5.

A ce propos, le procédé selon l'invention consiste en ce que l'on procède à un tel éloignement/rapprochement lorsque la température T5 du couvercle 5 et/ou la température T6 du fond 6 sont inférieures à une valeur déterminée (notamment inférieure à 110°C, de préférence inférieure à 100°, plus particulièrement comprise entre 90 et 100°C).

Un tel mode de réalisation permet, avantageusement, d'une part et en procédant à l'éloignement du fond 6, de détendre les déchets D (voire de faire sortir de l'air contenu dans les déchets D et/ou dans le sac S, ceci hors de ce sac S, notamment par aspiration) et, d'autre part et en procédant au rapprochement du fond 6, d'optimiser l'évacuation de l'air et de s'assurer qu'il n'y a plus d'air à l'intérieur de l'enceinte 3, du sac S et des déchets D, ceci avant d'appliquer le palier P de température.

En réduisant la quantité d'air dans l'enceinte, il est, avantageusement, remédié au moins aux problèmes de mauvais fonctionnement, d'inhomogénéité de la température et de mauvaise conduction de la température apparaissant lorsqu'un palier P de température est appliqué en présence d'air.

De manière surprenante, en procédant à un tel éloignement/rapprochement entre le fond 6 et le couvercle 5, on améliore substantiellement le traitement des déchets.

A ce propos, on observera que, selon un mode préféré de réalisation de l'invention, avant d'atteindre le palier P de température et après au moins un rapprochement entre le fond 6 et le couvercle 5 avec compression du sac S et des déchets D avec une pression déterminée (notamment supérieure à 120bars, de préférence de l'ordre de 140 bars), on relève, d'une part, la température (plus particulièrement la température T5 du couvercle 5 et/ou la température T6 du fond 6) et, d'autre part, la pression Pc au sein du sac S et des déchets D.

Le procédé consiste, alors, à procéder à un éloignement entre le fond 6 et le couvercle 5 suivi d'un nouveau rapprochement entre ce fond 6 et ce couvercle 5, ceci lorsque, d'une part, la température (T5 ; T6) est inférieure à une valeur déterminée (notamment inférieure à 110°C, de préférence inférieure à 100°C, plus particulièrement comprise entre 90 et 100°C) et, d'autre part, la pression Pc au sein du sac S et des déchets D est supérieure à une valeur déterminée (notamment supérieure à 80 bars, de préférence supérieure à 90 bars). En fait, dans ces conditions de température et de pression, il est estimé qu'il y a encore de l'air dans l'enceinte 3, le sac S et/ou les déchets D.

A ce propos, on observera que, lorsque, d'une part, la température (T5 ; T6) est inférieure à une valeur déterminée (notamment inférieure à 110°C, de préférence inférieure à 100°C, plus particulièrement comprise entre 90 et 100°C) et, d'autre part, la pression Pc au sein du sac S et des déchets D est inférieure à une valeur déterminée (notamment inférieure à 80 bars, de préférence inférieure à 60 bars), le procédé consiste à appliquer directement le palier P de température sans procéder à un éloignement /rapprochement du fond 6, ceci car il est estimé qu'il n'y a plus d'air dans l'enceinte 3, le sac S et/ou les déchets D.

Selon une autre caractéristique, après un nouveau rapprochement entre le fond 6 et le couvercle 5 (un tel nouveau rapprochement ayant suivi un éloignement entre ce fond 6 et ce couvercle 7), on relève la pression Pc au sein du sac S et des déchets D.

Dans un pareil cas, lorsque cette pression Pc est supérieure à une valeur déterminée de pression (notamment supérieure à 80 bars, de préférence supérieure à 90 bars), il est procédé à un nouvel éloignement/rapprochement du fond 6, voire à une interruption du procédé de traitement, ceci car il est estimé qu'il y a encore de l'air dans l'enceinte 3, le sac S et/ou les déchets D.

Selon un mode de réalisation préféré, on procède à une telle interruption lorsque, après une pluralité d'éloignements/rapprochements, la pression Pc est toujours supérieure à une valeur de pression déterminée (notamment supérieure à 80 bars, de préférence supérieure à 90 bars).

Cependant, lorsque cette pression Pc relevée est inférieure à une valeur déterminée de pression (notamment inférieure à 80 bars, de préférence inférieure à 60 bars), le procédé consiste à poursuivre la montée en température et à faire observer (au couvercle 5 et au fond 6) le palier P de température, ceci car il est estimé qu'il n'y a plus d'air dans l'enceinte 3, le sac S et/ou les déchets D.

Selon une caractéristique additionnelle, lorsque, après un éloignement du fond 6, on procède à un nouveau rapprochement de ce fond 6, on procède à un tel nouveau rapprochement en sorte que la distance entre ce fond 6 et ce couvercle 5, après ce nouveau rapprochement, soit supérieure ou égale à la distance entre ce fond 6 et ce couvercle 5 avant de procéder à l'éloignement entre ce fond 6 et ce couvercle 5.

A ce propos, on observera que l'éloignement du fond 6 est assuré sur une course comprise entre 2 et 10mm, de préférence de l'ordre de 4mm.

Tel que mentionné ci-dessus, on procède à un tel nouveau rapprochement en sorte que la distance entre ce fond 6 et ce couvercle 5, après ce nouveau rapprochement, soit supérieure ou égale à la distance entre ce fond 6 et ce couvercle 5 avant de procéder à l'éloignement entre ce fond 6 et ce couvercle 5.

A ce propos, on observera que cette distance est, en fait, déterminée en sorte que la charge (sac S et déchets D contenus dans ce sac S) soit en contact avec le couvercle 5 et avec le fond 6. Ceci permet une meilleure conduction de la chaleur entre, d'une part, le couvercle 5 et les déchets D et, d'autre part, le fond 6 et les déchets D, ce qui améliore grandement le traitement de ces déchets D. Cette distance peut, également, être déterminée pour que les déchets D soient détendus au sein du sac S et ne soient plus sous pression au moment d'appliquer le palier P de température. Cette caractéristique permet, de manière surprenante, d'améliorer le traitement des déchets.

Selon une autre caractéristique du procédé, avant de faire observer un palier P de température au couvercle 5 et au fond 6, on mesure la distance entre le fond 6 et le couvercle 5 pour, soit faire observer ledit palier P de température lorsque cette distance est inférieure à une valeur déterminée, soit interrompre le procédé lorsque cette distance est supérieure à cette valeur déterminée.

On observera que la valeur déterminée de cette distance est inférieure ou égale à 100mm, de préférence de l'ordre de 50mm.

Une telle caractéristique permet, avantageusement, de faire observer un palier P de température uniquement lorsque la distance est inférieure à une valeur déterminée, c'est-à-dire uniquement lorsque la charge (sac et déchets contenus dans ce sac) présente une épaisseur inférieure à une valeur déterminée. Cette caractéristique permet, avantageusement, d'améliorer la conduction de la chaleur et l'homogénéité de la température au sein de la charge et, ainsi, d'assurer un traitement approprié des déchets.

Une caractéristique additionnelle du procédé consiste en ce qu'on autorise l'évacuation de l'air contenu dans ce dispositif 1 (plus particulièrement contenu dans l'enceinte 3, le sac S et/ou les déchets D), ceci hors du dispositif 1, plus particulièrement hors de l'enceinte 3 que comporte ce dispositif 1.

En fait, on autorise l'évacuation de l'air par l'intermédiaire des moyens 9 d'évacuation de l'air mentionnés ci-dessus, ceci au moins pendant la montée en température et/ou au moins pendant au moins un rapprochement entre le couvercle 5 et le fond 6.

Cette caractéristique permet, là encore, d'améliorer la conduction de la chaleur et l'homogénéité de la température au sein de la charge.

Selon l'invention, on empêche l'évacuation de l'air pendant qu'on fait observer le palier P de température au couvercle 5 et au fond 6, ceci en refermant la vanne des moyens 9 pour évacuer l'air.

De plus, ce procédé consiste en ce qu'on assure une filtration de l'air évacué hors du dispositif 1, plus particulièrement hors de l'enceinte 3 que comporte ce dispositif 1. Une telle filtration est assurée par un filtre 90 que comporte le dispositif 1, plus particulièrement que comportent les moyens 9 pour évacuer l'air de ce dispositif 1.

Une caractéristique additionnelle consiste en ce qu'on assure le chauffage de ce filtre 90 à une température Tf permettant de vaporiser le liquide contenu dans l'air évacué.

A ce propos, on observera que cette température Tf est supérieure à 90 °C, de préférence supérieure à 140°C.

Un tel chauffage est, de préférence, assuré dès le démarrage du processus de traitement des déchets D conformément au procédé selon l'invention.

Un tel chauffage permet, avantageusement, de vaporiser le liquide (notamment l'eau) contenu dans l'air et, ainsi, d'éviter qu'un tel liquide pollue le filtre 90 et le rendre inefficace.

Une autre caractéristique du procédé consiste en ce qu'on détecte la présence d'un liquide (plus particulièrement au niveau des moyens 9 pour évacuer l'air) et que, en cas de détection d'un tel liquide, on empêche l'évacuation de l'air (en fermant la vanne des moyens 9 pour évacuer l'air) et/ou le rapprochement entre le fond 6 et le couvercle 5, voire on procède à l'interruption du processus de traitement des déchets D.

Tel que mentionné ci-dessus, l'air évacué est filtré par un filtre 90 chauffé ce qui réduit substantiellement la probabilité de détecter un tel liquide de sorte que la probabilité d'interruption du processus de traitement des déchets D conformément au procédé selon l'invention est considérablement réduite par rapport aux procédés de l'état de la technique. La mise en œuvre du procédé selon l'invention permet, alors avantageusement, d'augmenter la probabilité de mener le processus de traitement des déchets D jusqu'à son terme, ceci par rapport aux processus de l'état de la technique.

Tel que mentionné ci-dessus, le procédé consiste en ce qu'on fait observer un palier P de température au couvercle 5 et au fond 6. En fait, au cours de ce palier P de température, la température de ce couvercle 5 et la température de ce fond 6 sont comprises entre 140 et 160°C, de préférence de l'ordre de 150 °C. Un mode préféré de réalisation consiste en ce que, au cours de ce palier P de température, la température T5 de ce couvercle 5 et la température T6 de ce fond 6 sont comprises entre 155 et 158 °C, de préférence comprises entre 156 et 157°C.

En fait, un tel palier P de température est appliqué pendant une durée comprise entre 10 et 30 minutes, de préférence de l'ordre de 15 minutes.

A l'issue de ce palier P de température, le procédé consiste à calculer le delta de pression entre le début du chauffage et la fin du palier P de température. Lorsque ce delta est supérieur à une valeur déterminée (comprise entre 3, 5 et 5 bars, de préférence de l'ordre de 4,2 bars), les déchets D sont considérés comme étant traités. Cependant, si ce delta est inférieur à une telle valeur déterminée, les déchets sont considérés comme non traités. A ce propos, on observera que cette valeur déterminée de delta correspond, en fait, à la pression pour laquelle il se produit une rupture de l'ADN des déchets D.

Le procédé conforme à l'invention consiste en ce que, après avoir fait observer un palier P de température au couvercle 5 et au fond 6, il est procédé à un refroidissement de ce couvercle 5 et de ce fond 6, ceci par l'intermédiaire du moyen 12 pour refroidir ce couvercle 5 et ce fond 6.

De manière additionnelle, lorsque, au cours du refroidissement du couvercle 5 et du fond 6, la température de ce couvercle 5 et/ou de ce fond 6 atteint une valeur déterminée (comprise entre 80°C et 95°C, de préférence de l'ordre de 90°C), il peut être procédé à un rapprochement entre le fond 6 et le couvercle 5, plus particulièrement en assurant une compression de la charge, notamment sous une pression comprise entre 80 et 100 bars, de préférence de l'ordre de 90 bars. Ceci permet, avantageusement, d'obtenir une galette de déchets bien formée.

Le procédé consiste, ensuite, à poursuivre le refroidissement du couvercle 5 et du fond 6 pour, lorsque la température de ce couvercle 5 et/ou de ce fond 6 atteint une valeur déterminée (comprise entre 50°C et 70°C, de préférence de l'ordre de 60°C), procéder à l'ouverture de la vanne des moyens 9 pour évacuer l'air.

Finalement et pour une température du couvercle 5 et/ou du fond 6 déterminée (de préférence inférieure à 50°), il est procédé à l'ouverture du couvercle 5.

## Revendications

1. Procédé de traitement thermique de déchets (D), ce procédé consistant en ce que :
- on introduit les déchets (D) à traiter dans un sac (S);
- on positionne le sac (S) contenant les déchets (D) à l'intérieur d'un dispositif (1), conçu pour traiter ces déchets (D), et comportant un fût cylindrique (4), un couvercle (5) amovible de fermeture de ce fût cylindrique (4), et un fond (6) mobile à l'intérieur de ce fût cylindrique (4), ceci au moins en direction du couvercle (5);
- après positionnement du sac (S) contenant les déchets (D) à l'intérieur du dispositif (1):
- on procède à un traitement thermique de ces déchets (D), tout d'abord en assurant une montée en température du couvercle (5) et du fond (6) du dispositif (1) et, ensuite, en faisant observer à ce couvercle (5) et à ce fond (6) un palier (P) de température ; **caractérisé par le fait que** :
- au moins pendant la montée en température du couvercle (5) et du fond (6) et avant d'atteindre le palier (P) de température, on procède à au moins un rapprochement entre ce couvercle (5) et ce fond (6) ; et
- on confère au couvercle (5), respectivement au fond (6), une température qui diffère d'au plus de 2°C de la température du fond (6), respectivement du couvercle (5), ceci au moins durant le palier (P) de température.

2. Procédé de traitement thermique de déchets (D) selon la revendication 1, **caractérisé par le fait que**, lorsqu'on confère au couvercle (5), respectivement au fond (6), une température déterminée qui diffère d'au plus de 2°C de la température du fond (6), respectivement du couvercle (5) :
- on relève la température de ce couvercle (5), respectivement de ce fond (6);
- on chauffe ce couvercle (5), respectivement ce fond (6);
- on refroidit ce couvercle (5), respectivement ce fond (6) ;
- on pilote le chauffage de ce couvercle (5), respectivement de ce fond (6), ainsi que le refroidissement de ce couvercle (5), respectivement de ce fond (6), ceci au moins en fonction de la température déterminée et de la température relevée de ce couvercle (5), respectivement de ce fond (6).

3. Procédé de traitement thermique de déchets (D) selon l'une quelconque des revendications précédentes, **caractérisé par le fait que**, avant d'atteindre le palier (P) de température et après au moins un rapprochement entre le fond (6) et le couvercle (5), on procède à un éloignement entre ce fond (6) et ce couvercle (5) suivi d'un nouveau rapprochement entre ce fond (6) et ce couvercle (5).

4. Procédé de traitement thermique de déchets (D) selon l'une quelconque des revendications précédentes, **caractérisé par le fait que**, avant d'atteindre le palier (P) de température et après au moins un rapprochement entre le fond (6) et le couvercle (5) avec compression du sac (S) et des déchets (D) avec une pression déterminée, on relève, d'une part, la température et, d'autre part, la pression (Pc) au sein du sac (S) et des déchets (D) pour, lorsque la température est inférieure à une valeur déterminée et la pression (Pc) supérieure à une valeur déterminée, procéder à un éloignement entre le fond (6) et le couvercle (5) suivi d'un nouveau rapprochement entre ce fond (6) et ce couvercle (5).

5. Procédé de traitement thermique de déchets (D) selon l'une quelconque des revendications 3 ou 4, **caractérisé par le fait qu'**on procède à un nouveau rapprochement entre le fond (6) et le couvercle (5) en sorte que la distance entre ce fond (6) et ce couvercle (5), après ce nouveau rapprochement, soit supérieure ou égale à la distance entre ce fond (6) et ce couvercle (5) avant de procéder à l'éloignement entre ce fond (6) et ce couvercle (5).

6. Procédé de traitement thermique de déchets (D) selon l'une quelconque des revendications 3 à 5, **caractérisé par le fait que**, après le nouveau rapprochement entre le fond (6) et le couvercle (5), on relève la pression (Pc) au sein du sac (S) et des déchets (D) pour, soit poursuivre la montée en température et faire observer le palier (P) de température lorsque cette pression (Pc) relevée est inférieure à une valeur déterminée de pression, soit procéder à un nouvel éloignement/rapprochement entre le fond (6) et le couvercle (5) lorsque cette pression (Pc) est supérieure à cette valeur déterminée de pression, voire interrompre le procédé de traitement.

7. Dispositif (1) de traitement de déchets (D) comportant une enceinte (3), destinée à recevoir les déchets (D) à traiter, et comportant un fût cylindrique (4), un couvercle (5) amovible de fermeture de ce fût cylindrique (4), un fond (6) mobile à l'intérieur de ce fût cylindrique (4) ceci au moins en direction du couvercle (5), et au moins un moyen (8) pour chauffer ce couvercle (5), respectivement ce fond (6), ce dispositif (1) est **caractérisé par le fait qu'**il comporte des moyens pour conférer au couvercle (5), respectivement au fond (6), une température déterminée qui diffère d'au plus de 2°C de la température du fond (6), respectivement du couvercle (5), ces moyens pour conférer une température déterminée au couvercle (5), respectivement au fond (6), comportent :
- au moins un moyen (11) pour relever au moins la température de ce couvercle (5), respectivement de ce fond (6);
- au moins un moyen (12) pour refroidir ce couvercle (5), respectivement ce fond (6);
- des moyens (10) pour piloter le moyen (8) pour chauffer ce couvercle (5), respectivement ce fond (6), ainsi que le moyen (12) pour refroidir ce couvercle (5), respectivement ce fond (6), ceci au moins en fonction de la température déterminée et de la température relevée de ce couvercle (5), respectivement de ce fond (6).

8. Dispositif (1) de traitement de déchets (D) selon la revendication 7, **caractérisé par le fait que** les moyens pour conférer une température déterminée au couvercle (5), respectivement au fond (6), comportent au moins un moyen (11) pour relever la température du fond (6), respectivement du couvercle (5), ainsi qu'au moins un moyen (10) pour piloter le moyen (8) pour chauffer ce couvercle (5), respectivement ce fond (6), ainsi que le moyen (12) pour refroidir ce couvercle (5), respectivement ce fond (6), ce moyen (10) pour piloter étant conçu pour assurer un tel pilotage en fonction de la température relevée de ce fond (6), respectivement de ce couvercle (5).

9. Dispositif (1) de traitement de déchets (D) selon l'une quelconque des revendications 7 ou 8, **caractérisé par le fait que** le moyen (8) pour chauffer est constitué par une résistance électrique et que le moyen (12) pour refroidir est constitué par une conduite à l'intérieur de laquelle s'écoule un liquide caloriporteur.

10. Dispositif (1) de traitement de déchets (D) selon l'une quelconque des revendications 7 à 9, **caractérisé par le fait que** le couvercle (5), respectivement le fond (6), comporte un bloc thermique (13) incorporant au moins un moyen (8) pour chauffer ce couvercle (5), respectivement ce fond (6), ainsi qu'au moins un moyen (12) pour refroidir ce couvercle (5), respectivement de ce fond (6).

11. Dispositif (1) de traitement de déchets (D) selon la revendication 10, **caractérisé par le fait que** le bloc thermique (13) comporte une armature (14), noyée dans le matériau du bloc thermique (13), et sur laquelle reposent et/ou de laquelle sont rendus solidaires, au moins un moyen (8) pour chauffer et/ou au moins un moyen (12) pour refroidir.

12. Dispositif (1) de traitement de déchets (D) selon l'une quelconque des revendications 7 à 11, **caractérisé par le fait que** le moyen (8) pour chauffer et le moyen (12) pour refroidir s'étendent sensiblement dans un plan et sont conformés en sorte de définir au moins une boucle (15 ; 16) et que au moins une partie d'une boucle (15) du moyen (8) pour chauffer, respectivement au moins une partie d'une boucle (16) du moyen (12) pour refroidir, est inscrite à l'intérieur d'au moins une partie d'une boucle (16 ; 15) du moyen (12) pour refroidir, respectivement du moyen (8) pour chauffer.

13. Dispositif (1) de traitement de déchets (D) selon l'une quelconque des revendications 10 ou 11, **caractérisé par le fait que** le bloc thermique (13) comporte des moyens (17) pour positionner au moins un moyen (8) pour chauffer, respectivement au moins un moyen (12) pour refroidir, ceci par rapport à l'armature (14) et/ou par rapport à au moins un moyen (12) pour refroidir, respectivement par rapport à au moins un moyen (8) pour chauffer.

14. Dispositif (1) de traitement de déchets (D) selon l'une quelconque des revendications 10 ou 11, **caractérisé par le fait que** le couvercle (5), respectivement le fond (6), comporte une plaque (18) en matériau inoxydable, positionnée à l'intérieur du fut cylindrique (4), comportant une face interne orientée en direction du fond (6), respectivement du couvercle (5), ainsi qu'une face externe, orientée, d'une part, dans une direction opposée à celle du fond (6), respectivement du couvercle (5), et, d'autre part, en direction du bloc thermique (13) que comporte ce couvercle (5), respectivement ce fond (6), et qui est rendu solidaire de cette plaque (18) en matériau inoxydable.

15. Dispositif (1) de traitement de déchets (D) selon la revendication 14, **caractérisé par le fait que** le fond (6), respectivement le couvercle (5), comporte des moyens (19) pour la fixation de la plaque (18) en matériau inoxydable sur le bloc thermique (13) et/ou des moyens pour régler la position de cette plaque (18) en matériau inoxydable par rapport à ce bloc thermique (13).

## Patentansprüche

1. Verfahren zur Wärmebehandlung von Abfall (D), wobei dieses Verfahren darin besteht, dass:
- zu behandelnder Abfall (D) in einen Beutel (S) eingebracht wird;
- der Beutel (S), der den Abfall (D) enthält, im Inneren einer Vorrichtung (1) positioniert wird, die gestaltet ist, um diesen Abfall (D) zu behandeln, und eine zylindrische Hülse (4), einen abnehmbaren Verschlussdeckel (5) dieser zylindrischen Hülse (4) und einen beweglichen Boden (6) im Inneren dieser zylindrischen Hülse (4) mindestens in Richtung des Deckels (5) beinhaltet;
- nach dem Positionieren des Beutels (S), der den Abfall (D) enthält, im Inneren der Vorrichtung (1):
- eine Wärmebehandlung dieses Abfalls (D) vorgenommen wird, indem zuerst für einen Anstieg der Temperatur des Deckels (5) und des Bodens (6) der Vorrichtung (1) gesorgt wird, und danach dafür gesorgt wird, dass dieser Deckel (5) und dieser Boden (6) eine Temperaturstufe (P) einhalten; **dadurch gekennzeichnet, dass**:
- mindestens während des Anstiegs der Temperatur des Deckels (5) und des Bodens (6) und vor Erreichen der Temperaturstufe (P) mindestens eine Annäherung zwischen diesem Deckel (5) und diesem Boden (6) vorgenommen wird; und
- dem Deckel (5), beziehungsweise dem Boden (6) eine Temperatur verliehen wird, die sich höchstens um 2 °C von der Temperatur des Bodens (6), beziehungsweise des Deckels (5), mindestens während der Temperaturstufe (P), unterscheidet.

2. Verfahren zur Wärmebehandlung von Abfall (D) nach Anspruch 1, **dadurch gekennzeichnet, dass**, wenn dem Deckel (5), beziehungsweise dem Boden (6) eine bestimmte Temperatur verliehen wird, die sich um mehr als 2 °C von der Temperatur des Bodens (6), beziehungsweise des Deckels (5) unterscheidet:
- die Temperatur dieses Deckels (5), beziehungsweise dieses Bodens (6) festgestellt wird;
- dieser Deckel (5), beziehungsweise dieser Boden (6) erwärmt wird;
- dieser Deckel (5), beziehungsweise dieser Boden (6) abgekühlt wird;
- die Erwärmung dieses Deckels (5), beziehungsweise dieses Bodens (6), sowie die Abkühlung dieses Deckels (5), beziehungsweise dieses Bodens (6) mindestens in Abhängigkeit von der bestimmten Temperatur und der festgestellten Temperatur dieses Deckels (5), beziehungsweise dieses Bodens (6) angesteuert werden.

3. Verfahren zur Wärmebehandlung von Abfall (D) nach einem der vorstehenden Ansprüche, **dadurch gekennzeichnet, dass** vor Erreichen der Temperaturstufe (P) und nach mindestens einer Annäherung zwischen dem Boden (6) und dem Deckel (5) eine Entfernung zwischen diesem Boden (6) und diesem Deckel (5), gefolgt von einer erneuten Annäherung zwischen diesem Boden (6) und diesem Deckel (5), vorgenommen werden.

4. Verfahren zur Wärmebehandlung von Abfall (D) nach einem der vorstehenden Ansprüche, **dadurch gekennzeichnet, dass** vor Erreichen der Temperaturstufe (P) und nach mindestens einer Annäherung zwischen dem Boden (6) und dem Deckel (5) samt Komprimierung des Beutels (S) und des Abfalls (D) mit einem bestimmten Druck, einerseits die Temperatur und andererseits der Druck (Pc) innerhalb des Beutels (S) und des Abfalls (D) festgestellt werden, um, wenn die Temperatur niedriger als ein vorbestimmter Wert, und der Druck (Pc) höher als ein bestimmter Wert sind, eine Entfernung zwischen dem Boden (6) und dem Deckel (5), gefolgt von einer erneuten Annäherung zwischen diesem Boden (6) und diesem Deckel (5) vorgenommen werden.

5. Verfahren zur Wärmebehandlung von Abfall (D) nach einem der Ansprüche 3 oder 4, **dadurch gekennzeichnet, dass** eine erneute Annäherung zwischen dem Boden (6) und dem Deckel (5) vorgenommen wird, sodass der Abstand zwischen diesem Boden (6) und diesem Deckel (5) nach dieser erneuten Annäherung größer oder gleich dem Abstand zwischen diesem Boden (6) und diesem Deckel (5) ist, bevor die Entfernung zwischen diesem Boden (6) und diesem Deckel (5) vorgenommen wird.

6. Verfahren zur Wärmebehandlung von Abfall (D) nach einem der Ansprüche 3 bis 5, **dadurch gekennzeichnet, dass** nach der erneuten Annäherung zwischen dem Boden (6) und dem Deckel (5) der Druck (Pc) innerhalb des Beutels (S) und dem Abfall (D) festgestellt wird, um entweder den Anstieg der Temperatur fortzusetzen, und die Temperaturstufe (P) einhalten zu lassen, wenn dieser festgestellte Druck (Pc) niedriger als ein bestimmter Druckwert ist, oder eine erneute Entfernung/ Annäherung zwischen dem Boden (6) und dem Deckel (5) vorzunehmen, wenn dieser Druck (Pc) höher als dieser bestimmte Druckwert ist, oder gar das Behandlungsverfahren zu unterbrechen.

7. Vorrichtung (1) zur Behandlung von Abfall (D), umfassend eine Einschließung (3), die dazu bestimmt ist, den zu behandelnden Abfall (D) aufzunehmen, und eine zylindrische Hülse (4), einen abnehmbaren Verschlussdeckel (5) dieser zylindrischen Hülse (4) und einen beweglichen Boden (6) im Inneren dieser zylindrischen Hülse (4) mindestens in Richtung des Deckels (5) beinhaltend, und mindestens ein Mittel (8) zum Erwärmen dieses Deckels (5), beziehungsweise dieses Bodens (6), wobei diese Vorrichtung (1) **dadurch gekennzeichnet ist, dass** sie Mittel beinhaltet, um dem Deckel (5) beziehungsweise dem Boden (6) eine bestimmte Temperatur zu verleihen, die sich höchstens um 2 °C von der Temperatur des Bodens (6), beziehungsweise des Deckels (5) unterscheidet, wobei diese Mittel, um dem Deckel (5) beziehungsweise dem Boden (6) eine bestimmte Temperatur zu verleihen, Folgendes beinhalten:
- mindestens ein Mittel (11) zum Feststellen mindestens der Temperatur dieses Deckels (5), beziehungsweise dieses Bodens (6);
- mindestens ein Mittel (12) zum Abkühlen dieses Deckels (5), beziehungsweise dieses Bodens (6);
- Mittel (10) zum Ansteuern des Mittels (8) zum Erwärmen dieses Deckels (5), beziehungsweise dieses Bodens (6), sowie des Mittels (12) zum Abkühlen dieses Deckels (5), beziehungsweise dieses Bodens (6) mindestens in Abhängigkeit von der bestimmten Temperatur und der festgestellten Temperatur dieses Deckels (5), beziehungsweise dieses Bodens (6).

8. Vorrichtung (1) zur Behandlung von Abfall (D) nach Anspruch 7, **dadurch gekennzeichnet, dass** die Mittel, um dem Deckel (5) beziehungsweise dem Boden (6) eine bestimmte Temperatur zu verleihen, mindestens ein Mittel (11) beinhalten, um Temperatur des Bodens (6), beziehungsweise des Deckels (5) festzustellen, sowie mindestens ein Mittel (10), um das Mittel (8) zum Erwärmen dieses Deckels (5), beziehungsweise dieses Bodens (6), sowie das Mittel (12) zum Abkühlen dieses Deckels (5), beziehungsweise dieses Bodens (6) anzusteuern, wobei dieses Mittel (10) zum Ansteuern gestaltet ist, um für eine solche Ansteuerung in Abhängigkeit von der festgestellten Temperatur dieses Bodens (6), beziehungsweise dieses Deckels (5) zu sorgen.

9. Vorrichtung (1) zur Behandlung von Abfall (D) nach einem der Ansprüche 7 oder 8, **dadurch gekennzeichnet, dass** das Mittel (8) zum Erwärmen aus einem elektrischen Widerstand gebildet wird, und das Mittel (12) zum Abkühlen aus einer Leitung gebildet wird, im Inneren derer ein Wärmeträgerfluid strömt.

10. Vorrichtung (1) zur Behandlung von Abfall (D) nach einem der Ansprüche 7 bis 9, **dadurch gekennzeichnet, dass** der Deckel (5) beziehungsweise der Boden (6) einen Thermoblock (13) beinhaltet, der mindestens ein Mittel (8) zum Erwärmen dieses Deckels (5), beziehungsweise dieses Bodens (6), sowie ein Mittel (12) zum Abkühlen dieses Deckels (5), beziehungsweise dieses Bodens (6) beinhaltet.

11. Vorrichtung (1) zur Behandlung von Abfall (D) nach Anspruch 10, **dadurch gekennzeichnet, dass** der Thermoblock (13) eine Armatur (14) beinhaltet, die in das Material des Thermoblocks (13) eingelassen ist, und auf der mindestens ein Mittel (8) zum Erwärmen und/oder mindestens ein Mittel (12) zum Abkühlen aufliegen und/oder fest damit verbunden sind.

12. Vorrichtung (1) zur Behandlung von Abfall (D) nach einem der Ansprüche 7 bis 11, **dadurch gekennzeichnet, dass** sich das Mittel (8) zum Erwärmen und das Mittel (12) zum Abkühlen im Wesentlichen in einer Ebene erstrecken, und derart ausgeformt sind, um mindestens eine Schleife (15; 16) zu definieren, und dass mindestens ein Teil einer Schleife (15) des Mittels (8) zum Erwärmen, beziehungsweise mindestens ein Teil einer Schleife (16) des Mittels (12) zum Abkühlen im Inneren mindestens eines Teils einer Schleife (16; 15) des Mittels (12) zum Abkühlen, beziehungsweise des Mittels (8) zum Erwärmen eingetragen ist.

13. Vorrichtung (1) zur Behandlung von Abfall (D) nach einem der Ansprüche 10 oder 11, **dadurch gekennzeichnet, dass** der Thermoblock (13) Mittel (17) zum Positionieren mindestens eines Mittels (8) zum Erwärmen, beziehungsweise eines Mittels (12) zum Abkühlen in Bezug auf die Armatur (14) und/oder in Bezug auf mindestens ein Mittel (12) zum Abkühlen, beziehungsweise in Bezug auf mindestens ein Mittel (8) zum Erwärmen beinhaltet.

14. Vorrichtung (1) zur Behandlung von Abfall (D) nach einem der Ansprüche 10 oder 11, **dadurch gekennzeichnet, dass** der Deckel (5) beziehungsweise der Boden (6) eine Platte (18) aus nichtrostendem Material beinhaltet, die im Inneren der zylindrischen Hülse (4) positioniert ist, eine in Richtung des Bodens (6), beziehungsweise des Deckels (5) ausgerichtete Innenseite beinhaltend, sowie eine, einerseits in eine entgegengesetzte Richtung zu jener des Bodens (6), beziehungsweise des Deckels (5), und andererseits in Richtung des Thermoblocks (13), den dieser Deckel (5), beziehungsweise dieser Boden (6) beinhaltet ausgerichtete Außenseite, und die fest mit dieser Platte (18) aus nichtrostendem Material verbunden ist.

15. Vorrichtung (1) zur Behandlung von Abfall (D) nach Anspruch 14, **dadurch gekennzeichnet, dass** der Boden (6), beziehungsweise der Deckel (5) Mittel (19) zur Befestigung der Platte (18) aus nichtrostendem Material am Thermoblock (13) und/oder Mittel zum Einstellen der Position dieser Platte (18) aus nichtrostendem Material in Bezug auf diesen Thermoblock (13) beinhaltet.

## Claims

1. A method for thermal treating waste (D), this method consisting of:
- introducing the waste (D) to be treated in a bag (S);
- positioning the bag (S) containing the waste (D) inside a device (1), designed to treat this waste (D), and including a cylindrical barrel (4), a removable cover (5) for closing this cylindrical barrel (4), and a bottom (6) movable inside this cylindrical barrel (4), at least in the direction of the cover (5);
- after positioning the bag (S) containing the waste (D) inside the device (1):
- proceeding to a thermal treatment of this waste (D), first of all by ensuring a rise in the temperature of the cover (5) and of the bottom (6) of the device (1) and, then, by subjecting to this cover (5) and this bottom (6) a temperature level (P); **characterized by** the fact that:
- at least during the rise in temperature of the cover (5) and the bottom (6) and before reaching the temperature level (P), this cover (5) and this bottom (6) are at least brought closer, and
- a temperature which differs by at most 2 °C from the temperature of the bottom (6), respectively of the cover (5) is imparted to the cover (5), respectively the bottom (6), this at least during the temperature level (P).

2. The method for thermal treating waste (D) according to claim 1, **characterized by** the fact that, a determined temperature which differs by at most 2 °C of the temperature of the bottom (6), respectively of the cover (5) is imparted to the cover (5), respectively the bottom (6):
- the temperature of this cover (5), respectively of this bottom (6) is noted;
- this cover (5), respectively this bottom (6) is heated;
- this cover (5), respectively this bottom (6) is cooled;
- the heating of this cover (5), respectively of this bottom (6), as well as the cooling of this cover (5), respectively of this bottom (6), are controlled, this at least as a function of the determined temperature and of the temperature noted from this cover (5), respectively from this bottom (6).

3. The method for thermal treating waste (D) according to any one of the preceding claims, **characterized by** the fact that, before reaching the level (P) of temperature and after the bottom (6) and the cover (5) are at least brought closer, this bottom (6) and this cover (5) are moved away, then this bottom (6) and this cover (5) are brought closer again.

4. The method for thermal treating waste (D) according to any one of the preceding claims, **characterized by** the fact that, before reaching the level (P) of temperature and after the bottom (6) and the cover (5) are at least brought closer with compression of the bag (S) and the waste (D) with a determined pressure, on the one hand the temperature and on the other hand the pressure (Pc) within the bag (S) and waste (D) are noted for, when the temperature is lower than a determined value and the pressure (Pc) greater than a determined value, moving away the bottom (6) and the cover (5) then, bringing closer again this bottom (6) and this cover (5).

5. The method for thermal treating waste (D) according to any one of claims 3 or 4, **characterized by** the fact that the bottom (6) and the cover (5) are brought closer again so that the distance between this bottom (6) and this cover (5), after being brought closer again, is greater than or equal to the distance between this bottom (6) and this cover (5) before moving away this bottom (6) and this cover (5).

6. The method for thermal treating waste (D) according to any one of claims 3 to 5, **characterized by** the fact that, after bringing closer again the bottom (6) and the cover (5), the pressure (Pc) is noted within the bag (S) and the waste (D) to either continue the rise in temperature and observe the temperature level (P) when this noted pressure (Pc) is lower than a determined pressure value, or move away/bring closer the bottom (6) and the cover (5) again when this pressure (Pc) is greater than this determined pressure value, or even interrupt the treating method.

7. A device (1) for treating waste (D) including an enclosure (3), intended to receive the waste (D) to be treated, and including a cylindrical barrel (4), a cover (5) removable for closing this cylindrical barrel (4), a bottom (6) movable inside this cylindrical barrel (4) this at least in the direction of the cover (5), and at least one means (8) for heating this cover (5), respectively this bottom (6), this device (1) is **characterized by** the fact that it includes means for imparting the cover (5), respectively the bottom (6), a determined temperature which differs by at most 2 °C of the temperature of the bottom (6), respectively of the cover (5), these means for imparting a determined temperature to the cover (5), respectively to the bottom (6), include:
- at least one means (11) for noting at least the temperature of this cover (5), respectively of this bottom (6);
- at least one means (12) for cooling this cover (5), respectively this bottom (6);
- means (10) for controlling the means (8) for heating this cover (5), respectively this bottom (6), as well as the means (12) for cooling this cover (5), respectively this bottom (6), this at least as a function of the determined temperature and of the noted temperature of this cover (5), respectively of this bottom (6).

8. The device (1) for treating waste (D) according to claim 7, **characterized in that** the means for imparting a determined temperature to the cover (5), respectively to the bottom (6), include at least one means (11) to note the temperature of the bottom (6), respectively of the cover (5), as well as at least one means (10) for controlling the means (8) for heating this cover (5), respectively this bottom (6), as well as the means (12) for cooling this cover (5), respectively this bottom (6), this means (10) for controlling being designed to ensure such control as a function of the temperature measured from this bottom (6), respectively of this cover (5).

9. The device (1) for treating waste (D) according to any one of claims 7 or 8, **characterized by** the fact that the means (8) for heating is constituted by an electrical resistance and that the means (12) for cooling consists of a conduit inside which a heat-transfer liquid flows.

10. The device (1) for treating waste (D) according to any one of claims 7 to 9, **characterized by** the fact that the cover (5), respectively the bottom (6), includes a thermal block (13) incorporating at least one means (8) for heating this cover (5), respectively this bottom (6), as well as at least one means (12) for cooling this cover (5), respectively this bottom (6).

11. The device (1) for treating waste (D) according to claim 10, **characterized by** the fact that the thermal block (13) includes an armature (14), embedded in the material of the thermal block (13), and on which rest and/or which are made secured, at least one means (8) for heating and/or at least one means (12) for cooling.

12. The device (1) for treating waste (D) according to any one of claims 7 to 11, **characterized by** the fact that the means (8) for heating and the means (12) for cooling extend substantially in a plane and are shaped so as to define at least one loop (15; 16) and that at least one portion of a loop (15) of the means (8) for heating, respectively at least one portion of a loop (16) of the means (12) for cooling, is inscribed inside at least one portion of a loop (16; 15) of the means (12) for cooling, respectively of the means (8) for heating.

13. The device (1) for treating waste (D) according to any one of claims 10 or 11, **characterized by** the fact that the thermal block (13) includes means (17) for positioning at least one means (8) for heating, respectively at least one means (12) for cooling, this relative to the armature (14) and/or relative to at least one means (12) for cooling, respectively relative to at least one means (8) for heating.

14. The device (1) for treating waste (D) according to any one of claims 10 or 11, **characterized by** the fact that the cover (5), respectively the bottom (6), includes a plate (18) made of a stainless material, positioned inside the cylindrical barrel (4), including an inner face oriented towards the bottom (6), respectively of the cover (5), as well as an outer face, oriented, on the one hand, in a direction opposite to that of the bottom (6), respectively of the cover (5), and, on the other hand, in the direction of the thermal block (13) which includes this cover (5), respectively this bottom (6), and which is made secured to this plate (18) made of a stainless material.

15. The device (1) for treating waste (D) according to claim 14, **characterized by** the fact that the bottom (6), respectively the cover (5), includes means (19) for fixing the plate (18) made of a stainless material on the thermal block (13) and/or means for adjusting the position of this plate (18) made of a stainless material relative to this thermal block (13).
